# EUROPEAN PATENT APPLICATION

(11) **EP 4 749 649 A1**
(43) Date of publication of application: **27.05.2026**
(21) Application number: 24214771.8
(22) Date of filing: 22.11.2024
(51) Int. Cl.: G16H 30/40, G16H 50/20, G16H 50/70

(54) **CYSTOSCOPY SYSTEM AND DIAGNOSTIC METHOD FOR FLEXIBLE CYSTOSCOPY**

(71) Applicant: OLYMPUS WINTER & IBE GMBH, 22045 Hamburg (DE)
(72) Inventor: LAZZARI, Matteo, 22303 Hamburg (DE); MOMENI, Reza, 22527 Hamburg (DE)
(74) Representative: Seemann & Partner Patentanwälte mbB

(57) **Abstract**

The invention relates to a cystoscopy system 1 and a diagnostic method for flexible cystoscopy. The cystoscopy system 1 comprises a flexible cystoscope 3, a video processing unit 4, a user interface 5 and a computer based clinical decision support system 2. An AI model 24 is stored on the memory 21. The AI model 24 is trained on a dataset comprising multiple images of blood vessels 14 in bladder tissue. The cystoscope 3 captures video data 10 of tissue and transmits the video data 10 to the video processing unit 4. The video processing unit 4 processes the video data 10 and transmits the processed video data 10 to the processor 20. The processor 20 performs an inference operation in which the video data 10 is applied to the AI model 24 to detect abnormalities of blood vessels 14 in the video data 10 and highlight the abnormalities in the enhanced video data 11, 12. The enhanced video data 11, 12 is display on the user interface 5 to medical personnel.

## Description

The invention relates to a cystoscopy system, comprising a flexible cystoscope, a video processing unit, a user interface and a computer-based clinical decision support system. The invention further relates to a diagnostic method for flexible cystoscopy.

Cystoscopy is endoscopy of the urinary bladder utilizing a so called cystoscope. It is often used to detect certain medical conditions, for example infections or cancer.

In order to reduce the risk of recontamination of patients during the cystoscopy and/or to improve working efficiency, surgeons often deploy single-use cystoscopes, which are disposed after their use. However, the image quality of such single-use cystoscopes often lags behind the image quality of other endoscopes.

In addition, when treating a patient, it is often necessary to carry out multiple operations. For example, the medical personnel will conduct a diagnosis first. Afterwards, when the information gathered during the diagnosis have been studied, a treatment of any abnormalities detected during the diagnosis will follow. The gap between diagnosis and treatment may be days, weeks or even months. This greatly increases the waiting time for patients. Also, conducting two separate operations increases the risks, the discomfort for the patient and also the costs.

It is the object of the present invention to increase the patient comfort, diagnostic capabilities and overall quality of cystoscopies.

This object is achieved by a cystoscopy system, comprising a flexible cystoscope, a video processing unit, a user interface and a computer-based clinical decision support system, wherein the support system includes an input interface, a processor, a memory and an output interface, wherein an AI model is stored on the memory and the processor is configured to access the memory in order to run the AI model, wherein the AI model is trained on a dataset comprising multiple images of blood vessels in bladder tissue, including blood vessels in healthy bladder tissue and blood vessels in bladder tissue exhibiting abnormalities, wherein the cystoscope is configured to capture video data of tissue and transmit the video data to the video processing unit, wherein the video processing unit is configured to process the video data and transmit the processed video data to the processor via the input interface, wherein the processor is configured to perform an inference operation in which the video data is applied to the AI model to generate enhanced video data, wherein the AI model is trained to detect abnormalities of blood vessels in the video data and highlight the abnormalities in the enhanced video data, wherein the output interface is configured to transmit the enhanced video data to the user interface, wherein the user interface is configured to display the enhanced video data to medical personnel.

Advantageously, by utilizing the AI model trained on images of blood vessel patterns in bladder tissue, the cystoscopy system is able to identify abnormalities in the tissue indicating injuries or diseases, for example cancer. This helps the medical personnel during diagnosis, and in particular treatment, of the patient. By automatically highlighting any abnormalities, the AI model helps the medical personnel to quickly recognize those abnormalities, even if the image quality is not perfect.

The images used to train the AI model as well as video data captured by the cystoscope may comprise bladder tissue as well as tissue of adjacent regions. This increases the potential to detect abnormalities during the cystoscopy.

In particular, the AI model has been trained on an extensive data set comprising both bladder images and pathology reports. This endows the AI model with the capability to detect tissue abnormalities based on their structural and vascular patterns.

Preferably, the support system is configured to highlight the blood vessels in the enhanced video data, wherein in particular the support system is configured to highlight the blood vessels based on different preset visualization modes. Highlighting the blood vessels in the enhanced video data allows for a more clear differentiation of the individual blood vessels. This aids in a better identification of abnormalities, in particular inflammations. In particular, the blood vessels are highlighted in the video data before it is applied to the AI model. In this way, the AI model may identify and detect abnormalities better, even if the image quality of the video data is not as good as the images used to train the AI model.

Preferably, the support system is trained and configured to highlight the blood vessels by shifting a colour of the blood vessels in the enhanced video data to a darker colour. In other words, the colour of the blood vessels in the video data is changed to a darker colour in the enhanced video data. As the tissue of the bladder is usually of a lighter colour compared to the blood vessels, shifting the colour of the blood vessels to an even darker colour increases the contrast and thus the visibility of the blood vessels. This helps in guiding the medical personnel towards a more precise identification and in particular treatment.

According to an embodiment, the AI model is trained and configured to highlight the tissue abnormalities with a colour coded system using different coloured areas to designate a risk level of the abnormalities, wherein in particular the different coloured areas are green, yellow, orange and/or red rectangles. In other words, the cystoscopy system offers a dedicated graphical user interface, which maps critical areas with colour coded rectangles. For example, a low risk is indicated with a green rectangle, a higher risk area with a yellow rectangle, an even higher risk area with an orange rectangle and areas with the highest risk are indicated with red rectangle. The risk indicates the probability, that the area contains an abnormality. This makes the identification of abnormalities even easier for the medical personnel.

Preferably, the AI model is trained and configured to generate the enhanced video data in real-time. By generating the enhanced video data in real-time, the medical personnel is able to identify any abnormalities during the initial cystoscopy, making additional medical procedures redundant. In particular, the AI model continuously monitors the video data and promptly identifies any colour deviations indicative of bladder abnormalities.

Preferably, the AI model is trained and configured to recommend possible treatment options for detected abnormalities, in particular including laser therapy and/or injections. By recommending treatment options, it is possible to combine the diagnosis and treatment procedures, thus greatly reducing the time necessary for the treatment of abnormalities. The treatment options serve as a guideline for the medical personnel in order to choose the necessary treatment option and thus helps the medical personnel in their decision making process. In particular, the AI model offers tailored treatment recommendations based on the identified risk level. This further helps the medical personnel in finding the perfect treatment option for the patient. In particular, the treatment options are offered in real-time. This allows the medical personnel to choose and even conduct the treatment when carrying out the diagnosis.

Preferably, the cystoscope is a single-use cystoscope comprising a single-use video sensor. Single-use cystoscopes are configured and designed to be discarded after use. This is done in order to prevent recontamination of patients. However, the single-use video sensors of single-use cystoscopes often offer limited image quality compared to reusable cystoscopes. The use of the AI model in order to detect tissue abnormalities helps alleviate these deficiencies.

Preferably, the video processing unit is a portable unit and/or a table mounted unit. By designing the video processing unit as a portable unit and/or a table mounted unit, the versatility of the cystoscopy system is improved.

According to an embodiment, the support system is configured to establish a wireless connection to the video processing unit. By connecting the video processing unit wirelessly to the support system, it is not necessary to arrange the hardware of the support system in close contact to the video processing unit. For example, the support system may be arranged partly or completely in a different room or even a different building than the video processing unit. This allows the support system to use processors with a high computing capacity without obstructing the operation room.

Preferable, the support system comprises an AI cloud network or is connected to an AI cloud network. By performing the inference operation in an AI cloud, the performance can be advantageously increased dramatically. The AI cloud is in particular connected wirelessly to the video processing unit.

According to an embodiment, the support system is configured to perform a texture enhancement of the tissue in the enhanced video data. By enhancing the texture in the video data, the quality of the video data can be further improved.

Preferably, the support system is configured to perform a brightness enhancement separate to the texture enhancement and combine both enhancement to create an enhanced image. By combining brightness enhancement and texture enhancement, the image quality is greatly improved.

In particular, additional imaging modalities may be used to further enhance the texture structure details. In particular, the AI model is trained to build a colour map of tissue alterations using a specific colour scale. In this way, the visibility of tissue and blood vessel abnormalities can be further improved.

Preferably, the AI model is trained to generate a report, detailing the findings and providing recommendations for follow-up treatments or monitoring based on collected data and predicted outcomes. By generating a report, the AI model does not only provide real time feedback, but also summarizes any findings and important information of the procedure. This allows the medical personnel to study any abnormalities in detail even after the cystoscopy has concluded.

The object is further solved by a diagnostic method for flexible cystoscopy, wherein an AI model is trained on a dataset comprising multiple images of blood vessels in bladder tissue, including blood vessels in healthy bladder tissue and blood vessels in bladder tissue exhibiting abnormalities, wherein a cystoscope captures video data of tissue and transmits the video data to a video processing unit, wherein the video processing unit processes the video data and transmits the processed video data to a processor via an input interface, wherein the processor performs an inference operation in which the video data is applied to the AI model to detects abnormalities of blood vessels in the video data and highlight the abnormalities in the enhanced video data, wherein an output interface transmits the enhanced video data to a user interface, wherein the user interface displays the enhanced video data to medical personnel.

The diagnostic method embodies the same advantages and characteristics as the cystoscopy system describes above.

The diagnostic method is in particular not performed during the medical procedure itself.

Further characteristics of the invention will become apparent from the description of the embodiments according to the invention together with the claims and the included drawings. Embodiments according to the invention can fulfil individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text. The drawings show in:
- Fig. 1: a schematic simplified embodiment of a cystoscopy system,
- Fig. 2: a schematic simplified representation of unenhanced and enhanced video data, and
- Fig. 3: a schematic simplified representation of a method for enhancing the quality of video data during a cystoscopy.

In the drawings, the same or similar types of elements or respectively corresponding parts are provided with the same reference numbers in order to prevent the item from needing to be reintroduced.

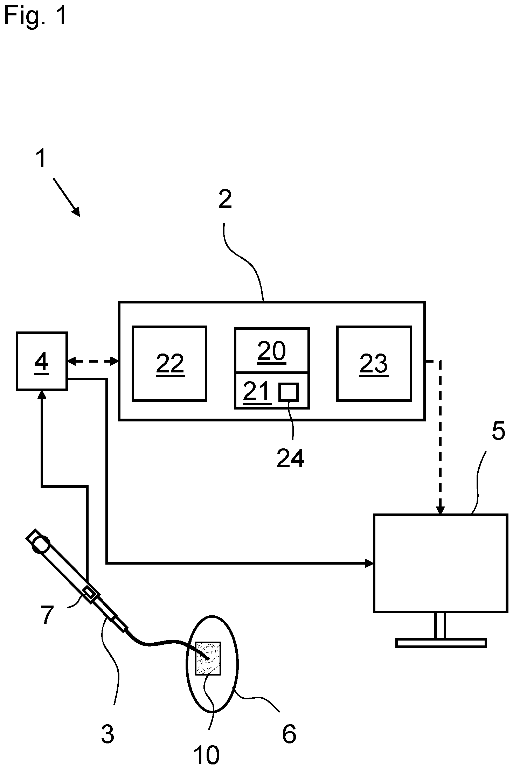

In fig. 1, the cystoscopy system 1 comprises a computer-based clinical decision support system 2, a cystoscope 3, a video processing unit 4 and a user interface 5. The cystoscope 3 captures video data 10 of blood vessels and tissue in a bladder 6 of a patient using a video sensor 7, for example a single-use video sensor 7. This video data 10 is transmitted to the video processing unit 4. The video processing unit 4 processes the video data 10 and transmits it to the support system 2. The support system 2 comprises a processor 20, a memory 21, an input interface 22 and an output interface 23. The input interface 22 is used to transmit the video data 10 from the video processing unit 4 to the processor 20 and/or the memory 21. In order to do so, the video processing unit 4 and the support system 2 may establish a wireless connection represented by a dashed line in fig. 1. For example, the support system 2 may comprise or be an AI cloud network. An AI model 24 is stored in the memory 21 of the support system 2. The processor 20 is configured to perform an inference operation in which the video data 10 is supplied to the AI model 24 in order to generate enhanced video data. In order to do so, the AI model 24 is trained on a data set comprising multiple images of blood vessels in a tissue of bladders 6. The AI model 24 is further trained to detect abnormalities in the blood vessels and enhance the video data 10 by highlighting these abnormalities. The enhanced video data is transmitted via the output interface 23 to a user interface 5. This may be done by transmitting the enhanced video data to the video processing unit 4, which transmits the enhanced video data to the user interface 5. The enhanced video data may also be transmitted to the user interface 5 directly from the output interface 23. In both cases, the transmission may be wireless. The user interface 5 may comprise a display, which displays the enhanced video data to the medical personnel.

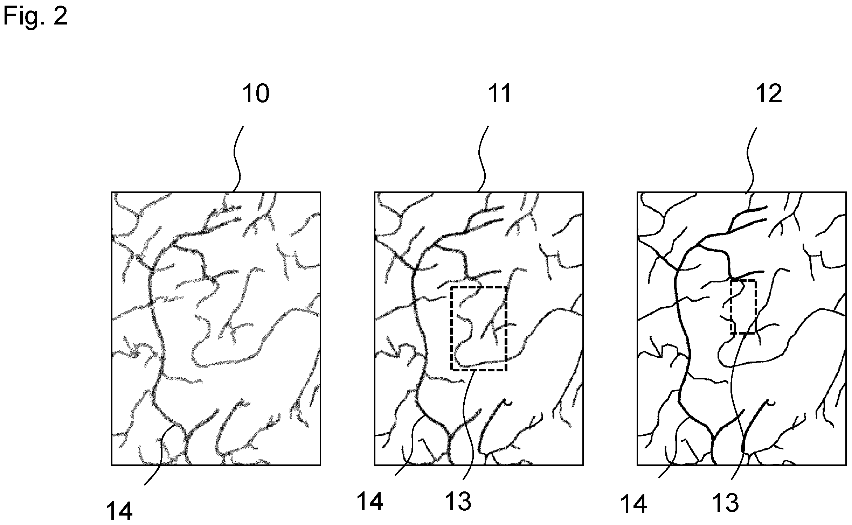

Fig. 2 shows schematic simplified representations of original video data 10 captured by the cystoscope 3 and enhanced video data 11, 12 created by the AI model 24. The video data 10 shows a number of blood vessels 14 in the tissue of the bladder 6 and its surrounding tissue. For clarity's sake, only one reference sign is assigned to the blood vessels 14. In the enhanced video data 11, 12, the quality of the video data 10 is improved. This may be done, for example, by highlighting the blood vessels 14, e.g. by shifting the colour of the blood vessels 14 to a darker colour. In addition, the texture and/or brightness of the video data 10 may be improved. The enhanced video data 11 and the enhanced video data 12 show different visualization modes, which offer help in the identification of different abnormalities.

In order to increase visibility of the abnormalities, the AI model 24 highlights the abnormalities with a colour coded system using coloured areas 13. These coloured areas 13 may take the form of rectangles in different colours, for example green, yellow, orange and/or red. A green rectangle may signify a low risk, whereas a red rectangle may signify a very high risk. This colour code gives the medical personnel a quick and easy to understand guide when viewing the enhanced video data 11, 12. The creation of the enhanced video data 11, 12 may take place in real-time, which makes it possible to not only diagnose abnormalities but also treat these abnormalities in the same procedure. In order to facilitate this, the AI model 24 may recommend treatment options.

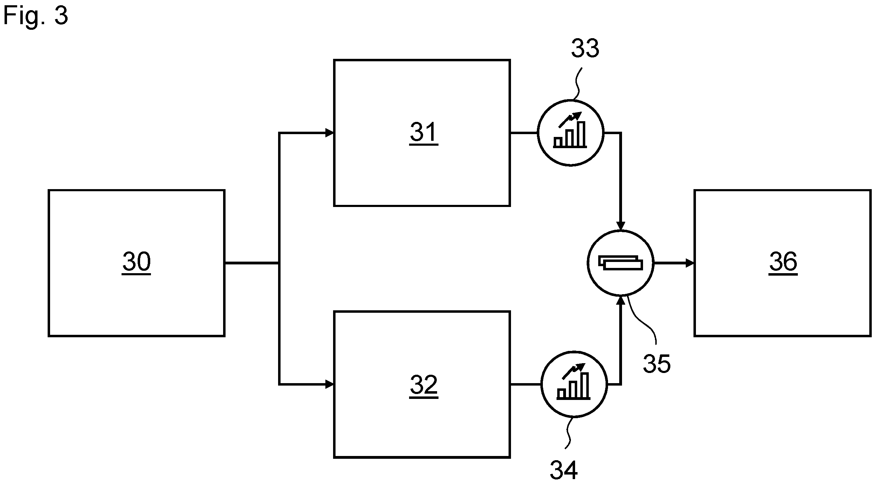

Fig. 3 shows a method for improving the image quality of the video data 10. An input image 30, which may be part of the video data 10, is split up into a texture image 31 and a brightness image 32. The texture image 31 is a black and white image, which facilitates a texture enhancement 33. The brightness image 32 is used to perform a brightness enhancement 34. The texture enhanced image and the brightness enhanced image are stacked to create an enhanced image 36. Due to the stacking 35, the resulting enhanced image 36 offers both an enhanced texture and an enhanced brightness, greatly increasing the quality of the image and visibility of the blood vessels 14.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

### List of References

- 1: cystoscopy system
- 2: support system
- 3: cystoscope
- 4: video processing unit
- 5: user interface
- 6: bladder
- 7: video sensor
- 10: video data
- 11: enhanced video data
- 12: enhanced video data
- 13: colored area
- 14: blood vessel
- 20: processor
- 21: memory
- 22: input interface
- 23: output interface
- 24: AI model
- 30: input image
- 31: texture image
- 32: brightness image
- 33: texture enhancement
- 34: brightness enhancement
- 35: stacking
- 36: enhanced image

## Claims

1. Cystoscopy system (1), comprising a flexible cystoscope (3), a video processing unit (4), a user interface (5) and a computer-based clinical decision support system (2), wherein the support system (2) includes an input interface (22), a processor (20), a memory (21) and an output interface (23), wherein an AI model (24) is stored on the memory (21) and the processor (20) is configured to access the memory (21) in order to run the AI model (24), wherein the AI model (24) is trained on a dataset comprising multiple images of blood vessels (14) in bladder tissue, including blood vessels (14) in healthy bladder tissue and blood vessels (14) in bladder tissue exhibiting abnormalities, wherein the cystoscope (3) is configured to capture video data (10) of tissue and transmit the video data (10) to the video processing unit (4), wherein the video processing unit (4) is configured to process the video data (10) and transmit the processed video data (10) to the processor (20) via the input interface (21), wherein the processor (20) is configured to perform an inference operation in which the video data (10) is applied to the AI model (24) to generate enhanced video data (11, 12), wherein the AI model (24) is trained to detect abnormalities of blood vessels (14) in the video data (10) and highlight the abnormalities in the enhanced video data (11, 12), wherein the output interface (23) is configured to transmit the enhanced video data (11, 12) to the user interface (5), wherein the user interface (5) is configured to display the enhanced video data (11, 12) to medical personnel.

2. Cystoscopy system (1) according to claim 1, wherein the support system (2) is configured to highlight the blood vessels (14) in the enhanced video data (11, 12), wherein in particular the support system (2) is configured to highlight the blood vessels (14) based on different preset visualization modes.

3. Cystoscopy system (1) according to claim 2, wherein the support system (2) is trained and configured to highlight the blood vessels (14) by shifting a colour of the blood vessels (14) in the enhanced video data (11, 12) to a darker colour.

4. Cystoscopy system (1) according to any one of claims 1 to 3, wherein the AI model (24) is trained and configured to highlight the tissue abnormalities with a colour coded system using different coloured areas (13) to designate a risk level of the abnormalities, wherein in particular the different coloured areas (13) are green, yellow, orange and/or red rectangles.

5. Cystoscopy system (1) according to any one of claims 1 to 4, wherein the AI model (24) is trained and configured to generate the enhanced video data (11, 12) in real-time.

6. Cystoscopy system (1) according to any one of claims 1 to 5, wherein the AI model (24) is trained and configured to recommend possible treatment options for detected abnormalities, in particular including laser therapy and/or injections.

7. Cystoscopy system (1) according to any one of claims 1 to 6, wherein the cystoscope (3) is a single-use cystoscope (3) comprising a single-use video sensor (7).

8. Cystoscopy system (1) according to any one of claims 1 to 7, wherein the video processing unit (4) is a portable unit and/or a table mounted unit.

9. Cystoscopy system (1) according to any one of claims 1 to 8, wherein the support system (2) is configured to establish a wireless connection to the video processing unit (4).

10. Cystoscopy system (1) according to any one of claims 1 to 9, wherein the support system (2) comprises an AI cloud network or is connected to an AI cloud network.

11. Cystoscopy system (1) according to any one of claims 1 to 10, wherein the support system (2) is configured to perform a texture enhancement (33) of the tissue in the enhanced video data (11, 12).

12. Cystoscopy system (1) according to any one of claims 1 to 11, wherein the support system (2) is configured to perform a brightness enhancement (34) separate to the texture enhancement (33) and combine both enhancement to create an enhanced image (36).

13. Cystoscopy system (1) according to any one of claims 1 to 12, wherein the AI model (24) is trained to generate a report, detailing the findings and providing recommendations for follow-up treatments or monitoring based on collected data and predicted outcomes.

14. Diagnostic method for flexible cystoscopy, wherein an AI model (24) is trained on a dataset comprising multiple images of blood vessels (14) in bladder tissue, including blood vessels (14) in healthy bladder tissue and blood vessels (14) in bladder tissue exhibiting abnormalities, wherein a cystoscope (3) captures video data (10) of tissue and transmits the video data (10) to a video processing unit (4), wherein the video processing unit (4) processes the video data (10) and transmits the processed video data (10) to a processor (20) via an input interface (22), wherein the processor (20) performs an inference operation in which the video data (10) is applied to the AI model (24) to detects abnormalities of blood vessels (14) in the video data (10) and highlight the abnormalities in the enhanced video data (11, 12), wherein an output interface (23) transmits the enhanced video data (11, 12) to a user interface (5), wherein the user interface (5) displays the enhanced video data (11, 12) to medical personnel.
